(19) Europäisches Patentamt / European Patent Office / Office européen des brevets

(11) EP 2 867 197 B1

(12) **FASCICULE DE BREVET EUROPEEN**

(45) Date de publication et mention de la délivrance du brevet:
**19.09.2018 Bulletin 2018/38**

(21) Numéro de dépôt: **13732908.2**

(22) Date de dépôt: **28.06.2013**

(51) Int Cl.:
***C07C 67/00*** *(2006.01)* ***C07C 69/716*** *(2006.01)*
***C10L 1/16*** *(2006.01)* ***C10L 1/19*** *(2006.01)*

(86) Numéro de dépôt international:
**PCT/EP2013/063593**

(87) Numéro de publication internationale:
**WO 2014/001486 (03.01.2014 Gazette 2014/01)**

(54) **PROCÉDÉ DE PRÉPARATION D'ESTERS DE L'ACIDE LÉVULINIQUE**

VERFAHREN ZUR HERSTELLUNG VON LEVULINSÄUREESTERN

METHOD FOR PREPARING LEVULINIC ACID ESTERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.06.2012 US 201261666294 P**
**02.07.2012 FR 1256334**

(43) Date de publication de la demande:
**06.05.2015 Bulletin 2015/19**

(73) Titulaires:
- **Centre National de la Recherche Scientifique (C.N.R.S.)**
  **75016 Paris (FR)**
- **Université Claude Bernard Lyon 1**
  **69100 Villeurbanne (FR)**

(72) Inventeurs:
- **ESSAYEM, Nadine**
  **F-38540 Saint Just Chaleyssin (FR)**
- **SAPALY, Gilbert**
  **F-69009 Lyon (FR)**
- **ETERNOT, Marion**
  **01480 Frans (FR)**
- **RATABOUL, Franck**
  **F-69008 Lyon (FR)**

(74) Mandataire: **Lavoix**
**62, rue de Bonnel**
**69448 Lyon Cedex 03 (FR)**

(56) Documents cités:
**WO-A1-03/085071**

- **FRANCK RATABOUL ET AL: "Cellulose Reactivity in Supercritical Methanol in the Presence of Solid Acid Catalysts: Direct Synthesis of Methyl-levulinate", INDUSTRIAL & ENGINEERING CHEMISTRY RESEARCH, vol. 50, no. 2, 19 janvier 2011 (2011-01-19), pages 799-805, XP055055197, ISSN: 0888-5885, DOI: 10.1021/ie101616e**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 867 197 B1

## Description

La présente invention concerne un procédé de préparation d'au moins un ester de l'acide lévulinique.

L'invention concerne également une composition comprenant au moins un ester de l'acide lévulinique et au moins une oléfine.

L'acide lévulinique, et tout particulièrement ses esters, sont des intermédiaires à très fort potentiel et représentent des produits clé au coeur des bioraffineries de demain.

Les esters de l'acide levulinique sont connus en tant qu'additifs carburants mais également en tant qu'intermédiaires de synthèse importants.

Plusieurs procédés de préparation d'esters de l'acide lévulinique ont été décrits.

WO 2005/070867 décrit un procédé utilisant une extraction réactive au cours duquel une solution aqueuse d'acide lévulinique, obtenue dans une première étape d'hydrolyse acide d'une biomasse, est mise en contact avec un alcool comportant au moins quatre atomes de carbone. Cet alcool, non miscible à l'eau agit comme réactif d'estérification mais aussi comme solvant d'extraction du levulinate.

WO 2003/085071 décrit l'obtention d'esters de l'acide lévulinique par estérification d'oléfines et de solutions aqueuses contenant de l'acide lévulinique. Ces solutions aqueuses d'acide lévulinique sont issues d'une première étape d'hydrolyse acide de biomasses diverses en présence d'eau et d'un catalyseur acide.

Les procédés de l'état de la technique comprennent nécessairement deux étapes dont une étape consistant en l'obtention d'une solution aqueuse d'acide lévulinique par hydrolyse acide d'une biomasse.

Toutefois, le recours à une telle étape entraîne différents problèmes, dont la génération d'éffluents aqueux acides, la difficulté du contrôle de la sélectivité de la transformation ou encore des problèmes de corrosion des installations.

Par ailleurs, un procédé de préparation de lévulinate de méthyle par réaction de cellulose avec du méthanol en conditions supercritiques en présence d'un catalyseur acide a été décrit (Rataboul et al, Ind.Eng.Chem.Res, 2011, vol. 50, n°2, pp. 799-805). Toutefois, le procédé décrit comprend la mise en oeuvre d'une phase liquide, nécessitant ainsi étape de séparation des phases afin de pouvoir récupérer le lévulinate de méthyle. De plus, la présence de méthanol génère la production d'eau, pouvant ainsi affecter l'activité catalytique du catalyseur acide.

Ainsi, un premier objectif de l'invention est de proposer un procédé de préparation d'esters de l'acide lévulinique qui apporte une solution à tout ou partie des problèmes des procédés de l'état de la technique.

Un autre objectif de l'invention est de proposer un procédé de préparation d'esters de l'acide lévulinique aisé à mettre en oeuvre, tout en ayant un rendement satisfaisant, et pouvant ainsi être transposé à une échelle industrielle.

Un autre objectif de l'invention est de proposer un procédé de préparation d'esters de l'acide lévulinique en l'absence d'eau ou en présence d'une faible quantité d'eau, permettant de limiter voire de supprimer l'obtention de sous-produits de réaction non souhaités, comme par exemple les humines.

Un autre objectif de l'invention est de proposer un procéder de préparation d'esters de l'acide lévulinique pouvant se présenter sous forme d'une composition liquide.

La présente invention a pour objet un procédé de préparation d'au moins un ester de l'acide lévulinique à partir d'une biomasse comprenant :

i) l'imprégnation de la biomasse au moyen d'un acide organique ou inorganique ;
ii) la mise en contact de la biomasse acidifiée avec un fluide supercritique comprenant au moins une oléfine.

Selon l'invention, un ester de l'acide lévulinique est un composé de formule (I)

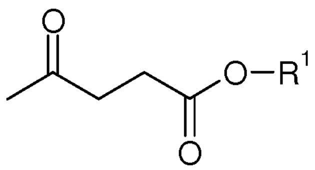

(I)

dans laquelle $R^1$ représente un groupement $C_2$-$C_{20}$ alkyle, ramifié ou linéaire, cyclique ou bicyclique.

De manière avantageuse, $R^1$ représente un groupement $C_2$-$C_6$ alkyle, linéaire ou ramifié.

De manière avantageuse, l'ester de l'acide lévulinique est choisi parmi le lévulinate d'éthyle, le lévulinate de propyle ou le lévulinate de butyle.

De manière encore plus avantageuse, l'ester de l'acide lévulinique est le lévulinate de butyle, notamment le

lévulinate de sec-butyle.

Par biomasse, on entend tout matériau comprenant au moins un carbohydrate.

Par biomasse, on entend également tout matériau comprenant un polysaccharide.

Selon l'invention, la biomasse peut être une biomasse lignocellulosique.

Le terme biomasse lignocellulosique (BLC) englobe plusieurs produits présents en quantités variables selon leur origine : la cellulose, l'hémicellulose, la lignine. L'hémicellulose et la cellulose constituent la partie carbohydrate de la lignocellulose. Ce sont des polymères de sucres (pentoses et hexoses). La lignine est une macromolécule riche en motifs phénoliques.

Selon l'invention, la biomasse lignocellulosique peut comprendre notamment du bois ou des déchets végétaux. D'autres exemples non limitatifs de matière biomasse lignocellulosique sont les résidus d'exploitation agricole, notamment de la paille, des herbes, des tiges, des noyaux, des coquilles, les résidus d'exploitation forestière, notamment des produits de première éclaircie, écorces, sciures, copeaux, chutes, les produits d'exploitation forestière, les cultures dédiées, notamment les taillis à courte rotation, les résidus de l'industrie agro-alimentaire, notamment les résidus de l'industrie du cotton, du bambou, du sisal, de la banane, du maïs, du *Panicum virgatum,* de l'alfalfa, de la noix de coco, de la bagasse, les déchets organiques ménagers, les déchets des installations de transformation du bois, les bois usagés de construction, du papier, recyclé ou non.

Selon l'invention, la biomasse lignocellulosique peut être utilisée sous sa forme brute, c'est-à-dire dans son intégralité de ces trois constituants : cellulose, hémicellulose et lignine. La biomasse brute se présente généralement sous forme de sciure ou de poudre. En général, elle est broyée ou déchiquetée pour permettre son transport.

Selon l'invention, la biomasse est de la cellulose et la taille moyenne de particules va de 1 à 50 $\mu$m, de préférence de 10 à 30 $\mu$m.

Selon l'invention, la biomasse est un bois résineux ou feuillu et la taille moyenne de particules va de 0,5 à 5 mm, de préférence de 1 à 2 mm. Comme exemple de bois, on peut citer l'épicéa.

De manière avantageuse, la biomasse se présente sous forme solide, notamment sous forme séchée ou déshydratée.

Selon l'invention, l'imprégnation de la biomasse est réalisée au moyen d'une solution aqueuse d'un acide organique ou inorganique.

Selon l'invention, l'acide organique peut être un acide carboxylique.

Selon l'invention, la nature de l'acide carboxylique doit être choisie pour pré-traiter la biomasse de façon satisfaisante.

Selon l'invention, la concentration du milieu en acide carboxylique doit être suffisamment importante pour pré-traiter la biomasse de façon satisfaisante.

En effet, généralement, la nature et la concentration du milieu en acides carboxyliques pouvant être libérés par la biomasse ne permettent pas d'obtenir une acidification suffisante de la biomasse nécessaire au traitement par un fluide supercritique comprenant au moins une oléfine.

Selon l'invention, l'acide peut être un acide inorganique.

Selon l'invention, l'acide inorganique peut être choisi parmi l'acide phosphorique, l'acide chlorhydrique ou l'acide sulfurique.

De manière avantageuse, l'acide inorganique est l'acide sulfurique.

Selon l'invention, l'imprégnation de la biomasse peut se faire dans des conditions d'imprégnation à humidité naissante.

A la différence d'une immersion complète de la biomasse dans la solution aqueuse d'acide organique ou inorganique, les conditions d'imprégnation à humidité naissante peuvent notamment correspondre à un volume de solution aqueuse d'acide proche ou équivalent au volume poreux de la biomasse à imprégner.

Selon l'invention, l'imprégnation de la biomasse peut se faire par imprégnation à sec ou par mise en contact de la biomasse avec une vapeur d'acide.

Généralement, selon l'invention, la durée d'imprégnation de la biomasse peut aller de quelques minutes à plusieurs heures, par exemple de 1 à 5 h.

De manière surprenante, il a été trouvé que la structure de la biomasse acidifiée du procédé selon l'invention n'est pas modifiée.

Ainsi, par exemple, la cellulose de la biomasse acidifiée présente les mêmes caractéristiques qu'une cellulose non imprégnée.

Le procédé selon l'invention permet ainsi d'obtenir l'ensemble de la biomasse disponible pour être mise en contact avec le fluide supercritique, en évitant les dégradations et/ou modifications de tout ou d'une partie de cette biomasse.

Selon l'invention, le fluide supercritique comprend une oléfine seule ou en mélange.

Selon l'invention, le fluide supercritique de l'étape ii) est obtenu par application de conditions de température et de pression proches ou supérieures à la température et à la pression critiques du fluide comprenant l'oléfine ou de l'oléfine en tant que fluide ou supérieures au point critique du fluide comprenant l'oléfine ou de l'oléfine en tant que fluide.

Ainsi, pour chaque oléfine seule ou en mélange, l'homme du métier pourra déterminer les conditions supercritiques de température et de pression du fluide.

Selon l'invention, le fluide supercritique comprend au moins un fluide organique.

De manière avantageuse, le fluide organique comprend au moins un composé organique.

Par oléfine, on entend tout composé de formule (II)

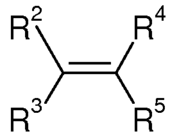

(II)

dans laquelle, $R^2$, $R^3$, $R^4$ et $R^5$, identiques ou différents, représentent indépendamment un hydrogène ou un groupement $C_1$-$C_6$ alkyle.

De manière avantageuse, le fluide supercritique comprend une oléfine choisi parmi l'éthylène, le propène ou le butène.

De manière encore plus avantageuse, le fluide supercritique comprend du butène, notamment du but-2-ène.

De manière avantageuse, le fluide supercritique est un fluide supercritique dense ou très dense.

De manière avantageuse, on applique une augmentation supplémentaire de la pression sur le fluide supercritique en vue d'en augmenter la densité.

L'homme du métier saura mettre en oeuvre les conditions de pression nécessaires à l'obtention d'un fluide supercritique dense ou très dense, notamment selon la nature du composé organique, par exemple selon l'oléfine mise en oeuvre.

Par exemple, pour le butène, on appliquera généralement une pression de 40 à 150 bars, de préférence de 50 à 100 bars.

Ainsi, par l'augmentation de la densité du fluide supercritique, on améliore le rendement du procédé selon l'invention.

Selon l'invention, une étape supplémentaire de séchage de la biomasse acidifiée peut être réalisée.

De manière avantageuse, le séchage est effectué à basse température, de préférence à température ambiante.

Ainsi, par ce séchage à basse température, les risques de dégradation de la biomasse sont limités, voire supprimés, au contraire d'un séchage par chauffage.

De manière avantageuse, le séchage du procédé selon l'invention peut être effectué sous vide ou à pression réduite.

Selon l'invention, le procédé peut être mis en oeuvre dans tout dispositif continu ou semi continu adapté à la réaction catalytique d'un composé en présence d'un fluide supercritique. Un exemple de dispositif est décrit dans Salinas et *al* (Salinas et al, Ing. Eng.Chem.Res, 2004, 43, 6355-6362).

Un autre objet de la présente invention concerne une composition comprenant au moins un ester de l'acide lévulinique et au moins une oléfine.

La composition selon l'invention se présente avantageusement sous forme liquide ou sous forme huileuse.

Les définitions et caractéristiques préférées de l'ester de l'acide lévulinique et de l'oléfine présentées pour le procédé selon l'invention s'appliquent à la composition selon l'invention.

Ainsi, l'ester de l'acide lévulinique est choisi parmi le lévulinate d'éthyle, le lévulinate de propyle ou le lévulinate de butyle.

De manière avantageuse, l'ester de l'acide lévulinique est le lévulinate de butyle, notamment le lévulinate de sec-butyle.

Ainsi, l'oléfine est choisie parmi l'éthylène, le propène ou le butène.

De manière avantageuse, l'oléfine est du butène, notamment du but-2-ène.

Selon l'invention, l'ester de l'acide lévulinique de la composition selon l'invention est issu d'une biomasse acidifiée par imprégnation de cette biomasse au moyen d'un acide organique ou inorganique suivie de la mise en contact avec un fluide supercritique comprenant au moins une oléfine.

En particulier, l'ester de l'acide lévulinique est de préférence préparé par la mise en oeuvre du procédé selon l'invention.

Les définitions et caractéristiques préférées de la biomasse et de l'acide présentées pour le procédé selon l'invention s'appliquent à la composition selon l'invention.

Ainsi, la biomasse peut être une biomasse lignocellulosique.

Le terme biomasse lignocellulosique (BLC) englobe plusieurs produits présents en quantités variables selon leur origine : la cellulose, l'hémicellulose, la lignine. L'hémicellulose et la cellulose constituent la partie carbohydrate de la lignocellulose. Ce sont des polymères de sucres (pentoses et hexoses). La lignine est une macromolécule riche en motifs phénoliques.

Selon l'invention, la biomasse lignocellulosique peut comprendre notamment du bois ou des déchets végétaux. D'autres exemples non limitatifs de matière biomasse lignocellulosique sont les résidus d'exploitation agricole, notamment de la paille, des herbes, des tiges, des noyaux, des coquilles, les résidus d'exploitation forestière, notamment des produits de première éclaircie, écorces, sciures, copeaux, chutes, les produits d'exploitation forestière, les cultures dédiées, notamment les taillis à courte rotation, les résidus de l'industrie agro-alimentaire, notamment les résidus de l'industrie du cotton, du bambou, du sisal, de la banane, du maïs, du *Panicum virgatum,* de l'alfalfa, de la noix de coco, de la bagasse, les déchets organiques ménagers, les déchets des installations de transformation du bois, les bois usagés de construction, du papier, recyclé ou non.

Selon l'invention, la biomasse lignocellulosique peut être utilisée sous sa forme brute, c'est-à-dire dans son intégralité de ces trois constituants : cellulose, hémicellulose et lignine. La biomasse brute se présente généralement sous forme de sciure ou de poudre. En général, elle est broyée ou déchiquetée pour permettre son transport.

Ainsi, l'acide organique peut être un acide carboxylique.

Ainsi, l'acide peut être un acide inorganique. Selon l'invention, l'acide inorganique peut être choisi parmi l'acide phosphorique, l'acide chlorhydrique ou l'acide sulfurique. De manière avantageuse, l'acide inorganique est l'acide sulfurique.

La composition selon l'invention peut avantageusement être utilisée comme base de carburant directement ou en mélange, par exemple comme additifs oxygénés pour carburants diesel ou essence.

Les différents aspects de l'invention sont illustrés par les exemples qui suivent. Ces exemples sont donnés à titre indicatif, sans caractère limitatif.

Dans ces exemples, l'oléfine est un mélange de but-2-éne cis et trans (45% cis et 55% cis), conditionnée dans une bouteille équipée d'un tube plongeur et pressurisée avec 8 bars d'He (Hélium).
Les coordonnées critiques du But-2-éne cis et du but-2-ène trans sont les suivants :

| | T critique (ºC) | P critique (bar) |
|---|---|---|
| trans-2-$C_4^=$ | 155.45 | 41.03 |
| cis-2-$C_4^=$ | 162.45 | 42.04 |

La réaction a eu lieu dans le dispositif tel que représenté dans la figure 1, comprenant :

1. four réactionnel,
2. réacteur tubulaire contenant un lit de biomasse solide,
3. une arrivée (bouteille avec tube plongeur et pompe) de l'oléfine liquide,
4. une alimentation en Helium,
5. un condenseur en aval du four réactionnel,
6. une régulation de pression.

La réaction a été mise en oeuvre dans un réacteur semi-continu dans lequel la biomasse était mise en contact avec l'oléfine en phase dense à haute température et haute pression. Par phase dense à haute température et haute pression, on entend des températures et pression supérieures, proches ou supérieures à la pression et à la température critiques de l'oléfine, de préférence une température comprise entre 150- 200°C et une pression comprise entre 40-150 bars.

Techniques de caractérisation : les produits liquides ont été analysés par chromatographie en phase gazeuse couplée à un spectromètre de masse.

Expression des résultats :

Le rendement en poids en sec-butyl lévulinate a été calculé selon l'équation suivante :

$$\text{Rdt en poids fr levulinate} = 100x(\text{m levulinate})/\text{m (carbohydrate sec)}$$

dans laquelle m (levulinate) représente la masse de sec-butyl levulinate produite et m (carbohydrate sec) représente la masse de biomasse sèche.

Le rendement molaire en sec-butyl levulinate (corrigé du nombre d'atomes de carbone) a été calculé selon l'équation suivante :

Rdt molaire en levulinate=100x(nb (mole levulinate)*5)/nb (mole UGP)

dans laquelle nb (mole levulinate) représente le nombre de moles de sec-butyl levulinate produites et nb (mole UGP) représente le nombre de moles d'unités glucose ou pentose (UGP) contenues dans la biomasse chargée dans le réacteur.

**Exemple 1 : préparation de sec levulinate de butyle à partir de cellulose ou d'épicéa et de but-2ène en conditions supercritiques**

La cellulose est une cellulose microcrystalline Sigmacell d'Aldrich,

La cellulose a été imprégnée de la façon suivante: 2 g de cellulose ont été imprégnés à humidité naissante avec une solution aqueuse :

- d'acide acétique (10 ou 30 % en poids) ; ou
- d'acide phosphorique (1% en poids) ; pu
- d'acide sulfurique (1% en poids).

La cellulose acidifiée a ensuite été séchée sous vide à température ambiante.

La cellulose acidifiée n'est pas affectée par cette imprégnation.
La figure 2 représente le spectre $^{13}C$ MAS-NMR de la cellulose non imprégnée.
La figure 3 représente le spectre $^{13}C$ MAS-NMR de la cellulose imprégnée au moyen d'une solution d'acide sulfurique à 1% en poids
La figure 4 représente montre le spectre $^{13}C$ MAS-NMR de la cellulose imprégnée au moyen d'une solution d'acide phosphorique à 1% en poids.
Ainsi, les trois spectres des figures 2, 3 et 4 sont identiques.
Ceci montre que le procédé d'imprégnation du procédé selon l'invention ne détériore ni ne modifie la structure de la biomasse utilisée, permettant ainsi que l'ensemble de cette biomasse soit disponible pour être mise en contact avec le fluide supercritique.

2g de cellulose acidifiée ont ensuite été placés dans un réacteur tubulaire. La biomasse a été maintenue dans la partie centrale du réacteur au moyen de tampons de laine de quartz. Les parties hautes et basses du réacteur ont été remplies par du carbure de silicium (dp=0.5mm).
Le réacteur a été connecté au test continu, pressurisé à la pression souhaitée au moyen d'hélium. Le but-2-ène liquide est pompé au moyen d'une pompe de type HPLC à un débit de 1cm$^3$/min afin de préalablement mouiller la biomasse puis le débit a été réduit à 0.1cm$^3$/min.
La température du réacteur a alors été progressivement augmentée jusqu'à la température de réaction.

Les produits liquides ont été récupérés en sortie de test à pression et température ambiantes. Après évaporation totale du but-2ène, l'échantillon liquide a été pesé et analysé par chromatographie en phase gazeuse.
Les résultats obtenus en fonction de la nature de l'acide utilisé lors du prétraitement et de la température de réaction sont rassemblés dans le tableau 1.

Tableau 1

| N° essai | Cellulose Prétraitement | T (°C) | P (bars) | liquide récupéré (g) | m solide récupéré (g) | Couleur Solide récupéré | Rdt sec-butyl Levulinate (% pds) | Rdt sec-butyl Levulinate (% mol) |
|---|---|---|---|---|---|---|---|---|
| 1 | sans | 170 | 100 | 0 | 2 | crème | - | - |
| 2 | $H_3PO_4$ (1% pds) | 170 | 100 | 0.23 | 1.35 | brun | 0.8 | 0.6 |
| 3 | $H_2SO_4$ (1% pds) | 150 | 100 | 0.8 | 0.94 | Brun foncé | 13.3 | 10.4 |
| 4 | $H_2SO_4$ (1% pds) | 170 | 100 | 1.1 | 0.94 | Brun foncé | 15.7 | 13.5 |
| 5 | $H_2SO_4$ (1% pds) | 170 | 50 | 0,17 | 1,19 | Brun foncé | 2.2 | 1.9 |

(suite)

| N° essai | Cellulose Prétraitement | T (°C) | P (bars) | liquide récupéré (g) | m solide récupéré (g) | Couleur Solide récupéré | Rdt sec-butyl Levulinate (% pds) | Rdt sec-butyl Levulinate (% mol) |
|---|---|---|---|---|---|---|---|---|
| 6 | $H_2SO_4$ (1% pds) | 170 | 30 | 0.02 | 1,15 | Brun foncé | 0.7 | 0.6 |
| 7 | $H_2SO_4$ (1% pds) | 130 | 100 | 0.09 | 1.25 | Brun foncé | 0.7 | 0.6 |
| % pds = pourcentage en poids | | | | | | | | |

Les résultats montrent qu'en l'absence de prétraitement (essai 1), la réaction conduite pendant 6h à 170°C et à 100 bars (température et pression supérieures à la température et à la pression critique du but-2-éne), aucun produit liquide à la pression et à la température ambiante n'est obtenu.
Ceci montre l'importance du prétraitement par imprégnation de la biomasse avec un acide organique ou inorganique.

Un traitement de la cellulose avec une solution aqueuse d'acide phosphorique à 1% suivi d'un traitement par un flux de but-2-ène à 170°C, permet de détecter des produits liquides en sortie de test. Plus d'un quart de la cellulose a été dépolymérisée. L'analyse des produits liquides récupérés par GC-SM montre la formation de sec-butyl levulinate, de sec-butyl formiate et de furfural comme produits majoritaires. Le rendement en sec-butyl lévulinate est de 0,6% (essai 2). La cellulose récupérée à l'issue de la réaction à 170°C a une coloration brune.
Le prétraitement de la cellulose par l'acide sulfurique, suivi de la réaction avec du but-2-ène à 150 ou 170 °C, 100 bars (essais 3 et 4), permet de dépolymériser la cellulose et produit une quantité significative de produits liquides à T et P ambiantes. Le rendement massique en sec-buty levulinate est de 15,7% (rendement molaire de 13,5%).
La cellulose prétraitée au moyen de l'acide sulfurique, suivie de la réaction avec du but-2-ène à 170°C mais à des pressions décroissantes 100 bars (essai 4 : oléfine phase SC haute pression), 50 bars (essai 5 : oléfine proche du point critique), 30 bars (essai 6 : oléfine phase gazeuse haute pression), produit des quantités décroissantes de produits liquides à température ambiante.
Le rendement le plus élevé est obtenu en faisant réagir la cellulose avec un flux d'oléfine en phase SC à haute pression, une phase SC dense (essai 4). Prêt du point critique (essai 5), la transformation de la cellulose est plus limitée tout comme le rendement en levulinate.
L'oléfine utilisée en phase gazeuse à même température (essai 6), ne permet pas la liquéfaction de la cellulose. Enfin, lorsque la cellulose, prétraitée par de l'acide sulfurique, est traitée sous un flux de butène liquide sous haute pression (essaie 7), la transformation de la cellulose en lévulinate n'est pas effective.
Ces essais montrent l'importance d'utiliser un flux de butène en phase supercritique, pour transformer la cellulose en levulinate en une seule étape.

**Exemple 2** : **préparation de sec levulinate de butyle à partir d'épicéa et de but-2-ène en conditions supercritiques**

L'épicéa a la composition suivante :

| Glucane | Xylane | Mannane | Galactane | Arabinane | Lignine |
|---|---|---|---|---|---|
| 46.2% | 8.2% | 14.2% | 2.5% | 1.2% | 26.1% |

Les prétraitements et la réaction ont été conduits comme dans l'exemple 1. L'épicéa a été broyé et tamisé.

Les résultats obtenus en fonction de la nature de l'acide utilisé lors du prétraitement et de la température de réaction sont rassemblés dans le tableau 2.

Tableau 2

| Essais | Epicéa Pré-traitement | T (°C) | P (bars) | m epicéa initiale (g) | m liquide récupéré (g) | m solide récupéré (g) | Couleur Solide récupéré | Rdt sec-butyl Levulinate (% pds) | Rdt sec-butyl Levulinate (% C) |
|---|---|---|---|---|---|---|---|---|---|
| 1 | sans | 170 | 100 | 1 | 0 | 1 | beige | - | - |
| 2 | $H_3PO_4$ (1%pds) | 170 | 100 | 1.24 | 0.34 | 0.78 | brun-noir | 3.4 | 2.8 |
| 3 | $H_2SO_4$ (1%pds) | 170 | 100 | 1.68 | 1.24 | 0.7 | noir | 8.8 | 7.7 |

% pds = pourcentage en poids

Sans prétraitement de l'épicéa, aucun produit liquide à P et T ambiantes n'est formé par réaction avec le sec-but-2ène en conditions SC (170°C, 100 bar), pendant 6h) (essai 1). L'épicéa récupéré après réaction est peu coloré, il a une légère teinte beige.
Suite à l'acidification de la biomasse d'épicéa par de l'acide phosphorique (1% en poids), la réaction avec le but-2-ène à 170°C permet une dépolymérisation de l'épicéa (essai 2). La biomasse solide a une couleur brun-noire après réaction. L'analyse du liquide formé montre un rendement en poids en sec-butyl levulinate de 3,4% calculé sur la partie carbohydrate de l'épicéa.
Suite à un prétraitement à l'acide sulfurique et réaction avec du but-2-ène en conditions supercritique, l'analyse du liquide récupéré, après évaporation à température et pression ambiantes, permet un rendement en sec-butyl levulinate de 8,8% (essai 3). La biomasse solide récupérée après réaction a une coloration noire.
Ces essais confirment l'importance du prétraitement de la biomasse par imprégnation avec un acide organique ou inorganique et ainsi que l'importance des conditions supercritiques du fluide comprenant l'oléfine.

**Revendications**

1. Procédé de préparation d'au moins un ester de l'acide lévulinique à partir d'une biomasse comprenant :

   i) l'imprégnation de la biomasse au moyen d'un acide organique ou inorganique ;
   ii) la mise en contact de la biomasse acidifiée avec un fluide supercritique comprenant au moins une oléfine.

2. Procédé selon la revendication 1 pour lequel la biomasse est une biomasse lignocellulosique.

3. Procédé selon la revendication 1 ou 2 pour lequel la biomasse se présente sous forme particulaire.

4. Procédé selon l'une quelconque des revendications précédentes pour lequel l'imprégnation de la biomasse se fait au moyen d'une solution aqueuse d'un acide organique ou inorganique.

5. Procédé selon l'une quelconque des revendications précédentes pour lequel l'acide est un acide inorganique, de préférence l'acide sulfurique.

6. Procédé selon l'une quelconque des revendications précédentes pour lequel l'imprégnation de la biomasse est effectuée dans des conditions d'imprégnation à humidité naissante.

7. Procédé selon l'une quelconque des revendications précédentes pour lequel le fluide supercritique est obtenu par application de conditions de température et de pression proches ou supérieures à la température et à la pression critiques du fluide comprenant au moins une oléfine ou de l'oléfine en tant que fluide.

8. Procédé selon l'une quelconque des revendications précédentes pour lequel le fluide supercritique est un fluide organique.

**9.** Procédé selon l'une quelconque des revendications précédentes pour lequel le fluide supercritique comprend du butène, de préférence du but-2-ène.

**10.** Procédé selon l'une quelconque des revendications précédentes pour lequel le fluide supercritique est un fluide supercritique dense ou très dense.

**11.** Procédé selon l'une quelconque des revendications précédentes comprenant également le séchage de la biomasse acidifiée.

**12.** Procédé selon la revendication précédente pour lequel le séchage est effectué à basse température, de préférence à température ambiante.

## Patentansprüche

**1.** Verfahren zur Herstellung mindestens eines Esters der Levulinsäure aus einer Biomasse, umfassend:

i) Imprägnieren der Biomasse mit einer organischen oder anorganischen Säure;
ii) Inkontaktbringen der gesäuerten Biomasse mit einem überkritischen Fluid, das mindestens ein Olefin enthält.

**2.** Verfahren nach Anspruch 1, bei dem die Biomasse eine Biomasse aus Lignocellulose ist.

**3.** Verfahren nach Anspruch 1 oder 2, bei dem die Biomasse in partikulärer Form vorliegt.

**4.** Verfahren nach einem beliebigen der vorhergehenden Ansprüche, bei dem das Imprägnieren der Biomasse mittels einer wässrigen Lösung einer organischen oder anorganischen Säure durchgeführt wird.

**5.** Verfahren nach einem beliebigen der vorhergehenden Ansprüche, bei dem die Säure eine anorganische Säure, vorzugsweise eine Schwefelsäure ist.

**6.** Verfahren nach einem beliebigen der vorhergehenden Ansprüche, bei dem das Imprägnieren der Biomasse bei Bedingungen beginnender Feuchtigkeit durchgeführt wird.

**7.** Verfahren nach einem beliebigen der vorhergehenden Ansprüche, bei dem das überkritische Fluid durch Anwenden von Temperatur- und Druckzuständen erhalten wird, die nahe oder größer als die Temperatur und der kritische Druck des Fluids sind, das mindestens ein Olefin oder das Olefin als Fluid enthält.

**8.** Verfahren nach einem beliebigen der vorhergehenden Ansprüche, bei dem das überkritische Fluid ein organisches Fluid ist.

**9.** Verfahren nach einem beliebigen der vorhergehenden Ansprüche, bei dem das überkritische Fluid Buten, vorzugsweise But-2-en enthält.

**10.** Verfahren nach einem beliebigen der vorhergehenden Ansprüche, bei dem das überkritische Fluid ein dichtes oder sehr dichtes überkritisches Fluid ist.

**11.** Verfahren nach einem beliebigen der vorhergehenden Ansprüche, umfassend auch die Trocknung der gesäuerten Biomasse.

**12.** Verfahren nach dem vorhergehenden Anspruch, für das die Trocknung bei tiefer Temperatur, vorzugsweise bei Umgebungstemperatur durchgeführt wird.

## Claims

**1.** A method for preparing at least one ester of levulinic acid from a biomass comprising:

i) impregnation of the biomass by means of an organic or inorganic acid;

ii) putting the acidified biomass in contact with a supercritical fluid comprising at least one olefin.

**2.** The method according to claim 1, for which the biomass is a lignocellulose biomass.

**3.** The method according to the invention 1 or 2, for which the biomass appears in a particulate form.

**4.** The method according to any of the preceding claims for which the impregnation of the biomass is accomplished by means of an aqueous solution of an organic or inorganic acid.

**5.** The method according to any of the preceding claims for which the acid is an inorganic acid, preferably sulfuric acid.

**6.** The method according to any of the preceding claims for which the impregnation of the biomass is carried out under impregnation conditions with nascent humidity.

**7.** The method according to any of the preceding claims, for which the supercritical fluid is obtained by applying temperature and pressure conditions close to or greater than the critical temperature and pressure of the fluid comprising at least one olefin or of the olefin as a fluid.

**8.** The method according to any of the preceding claims, for which the supercritical fluid is an organic fluid.

**9.** The method according to any of the preceding claims, for which the supercritical fluid comprises butene, preferably but-2-ene.

**10.** The method according to any of the preceding claims, for which the supercritical fluid is a dense or very dense supercritical fluid.

**11.** The method according to any of the preceding claims also comprising the drying of the acidified biomass.

**12.** The method according to the preceding claim, for which the drying is carried out at low temperature, preferably at room temperature.

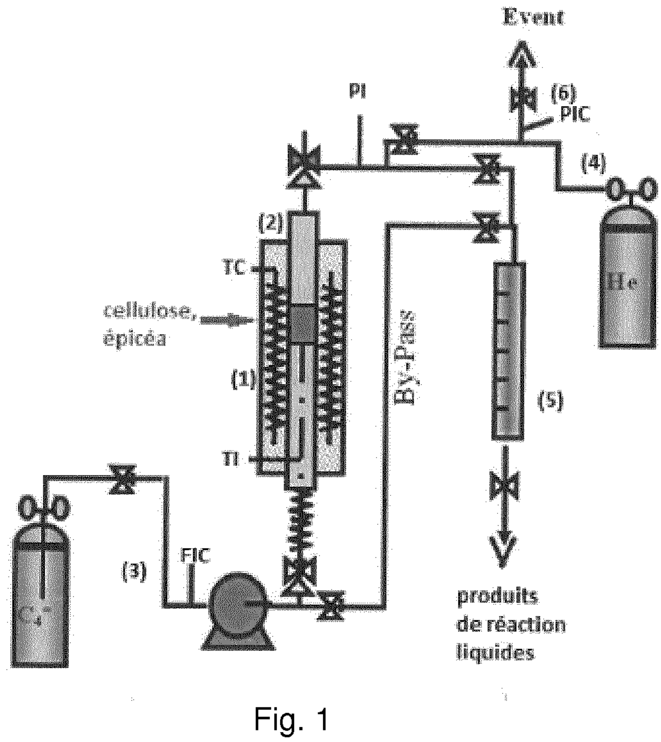
Event
(6)
PIC
PI
(4)
(2)
TC
cellulose,
épicéa
He
By-Pass
(1)
(5)
TI
FIC
(3)
produits
de réaction
liquides

Fig. 1

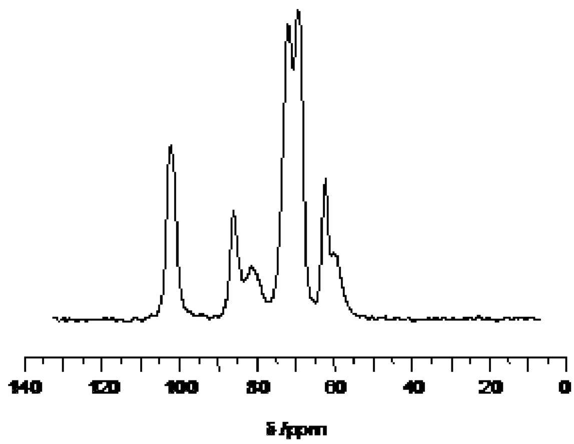
140
120
100
80
60
40
20
0
δ /ppm

Fig. 2

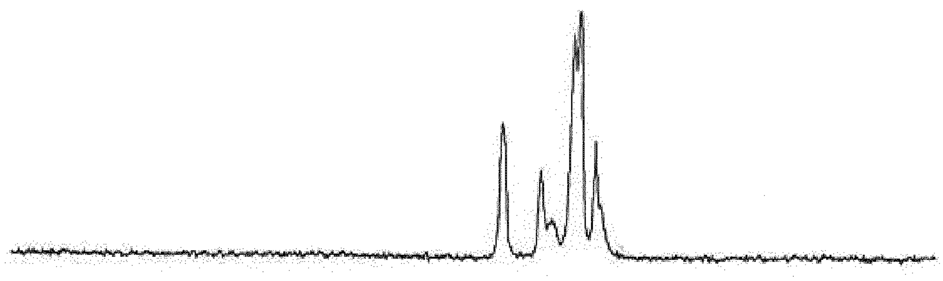

Fig. 3

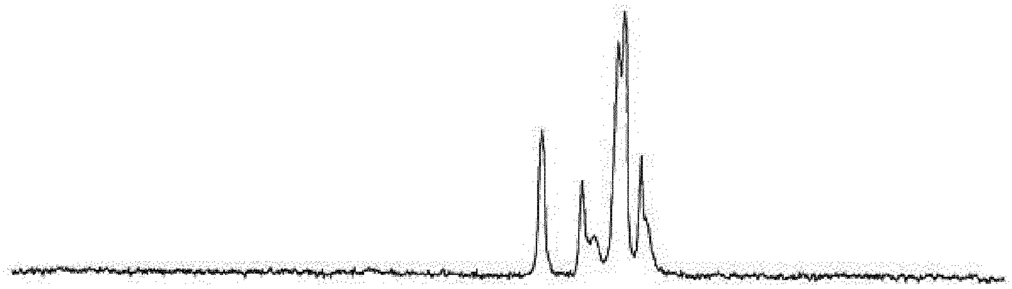

Fig. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*


### Documents brevets cités dans la description

- WO 2005070867 A
- WO 2003085071 A

### Littérature non-brevet citée dans la description

- **RATABOUL et al.** *Ind.Eng.Chem.Res,* 2011, vol. 50 (2), 799-805
- **SALINAS et al.** *Ing. Eng.Chem.Res,* 2004, vol. 43, 6355-6362